# FASCICULE DE BREVET EUROPEEN

(11) **EP 2 872 182 B1**
(45) Date de publication et mention de la délivrance du brevet: **20.03.2019**
(21) Numéro de dépôt: 13766397.7
(22) Date de dépôt: 12.07.2013
(51) Int. Cl.: A61K 33/44, A61P 35/00, A61N 5/10, A61K 41/00

(54) **UTILISATION DE NANODIAMANTS POUR GENERER DES RADICAUX LIBRES A VISEE THERAPEUTIQUE SOUS IRRADIATION**
VERWENDUNG VON NANODIAMANTEN ZUR ERZEUGUNG FREIER RADIKALE FÜR THERAPEUTISCHE ZWECKE UNTER BESTRAHLUNG
USE OF NANODIAMONDS FOR GENERATING FREE RADICALS FOR THERAPEUTIC PURPOSES UNDER RADIATION

(30) Priorité: 13.07.2012 FR 1256786; 13.07.2012 FR 1256781; 16.11.2012 FR 1260924
(43) Date de publication de la demande: 20.05.2015
(73) Titulaire: Commissariat à l'Énergie Atomique et aux Énergies Alternatives, 75015 Paris (FR)
(72) Inventeur: PETIT, Tristan, F-75011 Paris (FR); ARNAULT, Jean-Charles, F-78120 Rambouillet (FR); GIRARD, Hugues, F-78220 Viroflay (FR); GRALL, Romain, 21000 Dijon (FR); CHEVILLARD, Sylvie, F-75005 Paris (FR); DELIC, Jozo, F-92340 Bourg La Reine (FR)
(74) Mandataire: Gevers & Orès
(86) Numéro de dépôt international: PCT/IB2013/055766
(87) Numéro de publication internationale: WO 2014/009930

(56) Documents cités:
- US-A1- 2011 008 447
- KUZNETSOV OLEKSANDR ET AL: "Water-soluble nanodiamond.", LANGMUIR : THE ACS JOURNAL OF SURFACES AND COLLOIDS 20 MAR 2012, vol. 28, no. 11, 20 mars 2012 (2012-03-20) , pages 5243-5248, XP002697335, ISSN: 1520-5827
- CHI-CHENG FU ET AL: "Characterization and application of single fluorescent nanodiamonds as cellular biomarkers", PROCEEDINGS OF THE NATIONAL ACADEMY OF SCIENCES, NATIONAL ACADEMY OF SCIENCES, US, vol. 104, no. 3, 16 janvier 2007 (2007-01-16), pages 727-732, XP002456036, ISSN: 0027-8424, DOI: 10.1073/PNAS.0605409104
- GIRARD H A ET AL: "Surface properties of hydrogenated nanodiamonds: a chemical investigation.", PHYSICAL CHEMISTRY CHEMICAL PHYSICS : PCCP 28 JUN 2011, vol. 13, no. 24, 28 juin 2011 (2011-06-28) , pages 11517-11523, XP002697336, ISSN: 1463-9084
- ARNAULT JEAN-CHARLES ET AL: "Surface chemical modifications and surface reactivity of nanodiamonds hydrogenated by CVD plasma.", PHYSICAL CHEMISTRY CHEMICAL PHYSICS : PCCP 28 JUN 2011, vol. 13, no. 24, 28 juin 2011 (2011-06-28) , pages 11481-11487, XP002697337, ISSN: 1463-9084

## Description

La présente invention concerne le domaine de la radiothérapie. Elle a notamment pour objet l'utilisation de nanoparticules de diamant particulières (ou nanodiamants) comme médicament, en combinaison avec un rayonnement ionisant, pour générer localement des radicaux libres et détruire des cellules cibles.

La radiothérapie est une technique courante pour traiter des tumeurs cancéreuses, utilisée dans environ 50% des cas, Elle consiste à créer des radicaux libres dans les cellules par irradiation localisée ; ces radicaux libres provoquent des ruptures de l'ADN des cellules irradiées, conduisant à leur mort. L'efficacité des traitements par radiothérapie est actuellement limitée par la résistance de certaines tumeurs aux rayonnements ionisants, par rapport aux cellules saines. Une radiosensibilisation sélective et efficace des cellules tumorales permettrait d'améliorer significativement l'efficacité de ces traitements et de réduire les effets secondaires sur les tissus sains.

Dans ce but, différentes approches ont été décrites, telles que l'utilisation de nanoparticules capables de générer localement des radicaux libres, ou l'utilisation de radiosensibilisants.

La première approche consiste à générer localement, au sein de la tumeur, des radicaux libres en utilisant des nanoparticules. Les méthodes de ce type décrites à ce jour sont basées sur des propriétés physiques, liées aux nanoparticules, permettant de générer efficacement des espèces radicalaires à leur surface sous irradiation. Les nanoparticules utilisées sont généralement constituées d'atomes ayant un numéro atomique (Z) élevé, afin d'absorber plus efficacement les rayons X, mais qui sont généralement des matériaux coûteux (or, platine, terres rares), et/ou pouvant induire une toxicité, et/ou peu stables dans un milieu biologique. A titre d'exemple, on peut citer la demande de brevet US2008/0003183 (Ting Guo), qui propose d'utiliser des nanoparticules constituées d'éléments lourds comme l'or, capables d'émettre des électrons Auger localement sous irradiation. Cette génération d'électrons peut être induite en utilisant des rayons X ayant une énergie que les molécules d'eau n'absorbent que faiblement, afin de générer les radicaux libres essentiellement à proximité des nanoparticules. Cependant, pour améliorer la stabilité colloïdale et la biocompatibilité de ces nanoparticules, le greffage de molécules est souvent nécessaire, ce qui peut atténuer la dose d'électrons secondaires ou Auger transmis à l'environnement de la nanoparticule, et réduit donc la dose de radicaux libres générés.

Selon la deuxième approche citée plus haut, de nouvelles molécules radiosensibilisantes sont actuellement en cours d'étude, dont le but est de cibler les défenses biologiques propres aux cellules tumorales (C. Begg *et al,* 2011). Malheureusement, ces molécules ne peuvent pas toujours être délivrées dans les cellules tumorales *in vivo,* ce qui limite leur utilisation thérapeutique. C'est notamment le cas des ARNs interférents de *POLQ,* qui ont montré récemment une grande sélectivité dans la radiosensibilisation de cellules tumorales *in vitro* (Higgins *et al,* 2010). Ces ARNs interférents pourront difficilement être utilisés *in vivo* sans moyen de vectorisation, car leur biodisponibilité est limitée.

Les nanodiamants peuvent être produits de façon à être solubles dans l'eau (Kuzntsov *et al.,* 2012).Différentes applications biotechnologiques sont actuellement connues pour les nanodiamants, telles que la vectorisation et la délivrance de médicaments et d'ARN interférents dans les cellules tumorales. Dans ces applications, les nanodiamants sont utilisés uniquement comme vecteurs passifs. A titre d'exemple, on peut citer la demande de brevet US 2010/0305309 (Ho et al.), portant sur différents procédés pour délivrer des médicaments avec des nanodiamants. Dans ce cas particulier, les nanodiamants utilisés comme vecteurs sont oxydés en surface, ce qui leur donne une charge de surface négative et rend nécessaire l'ajout de polymères pour permettre de vectoriser des brins d'ADN ou d'ARN ayant aussi une charge négative. Ces polymères peuvent induire une toxicité supplémentaire et augmentent significativement la taille des nanodiamants, ce qui peut induire une plus grande rétention dans les organes comme le foie ou les reins pour des applications *in* vivo. Un autre exemple d'utilisation des nanodiamants comme vecteurs passifs est décrit dans la demande US 2011/10008447 (Jui-I Chao et al.). Des nanodiamants modifiés, possédant des propriétés fluorescentes ont également été décrits pour leur utilisation comme biomarqueurs cellulaires (Chi-Cheng Fu *et al.,* 2007).

La présente invention repose sur la mise en évidence, par les inventeurs, d'une propriété jusqu'ici ignorée des nanodiamants. Le terme "nanodiamant" désigne ici toute nanoparticule principalement constituée d'atomes de carbone hybridé sp³, ayant une taille inférieure à 250 nm. Ces nanoparticules peuvent être, entre autres, synthétisées par broyage de diamant de synthèse ou par détonation. Dans ce dernier cas, les nanodiamants ont généralement une taille inférieure à 10 nm. Le terme "nanodiamant" désigne également des agrégats de taille nanoparticulaire constitués de nanoparticules de diamant primaires. Les inventeurs ont observé que les nanodiamants ont des propriétés physiques particulières qui leur permettent de générer efficacement des radicaux libres sous rayonnement, notamment sous rayonnement ionisant. Les nanodiamants sont en effet constitués d'une très grande concentration d'atomes de carbone (1.8 × 10²³ atomes/cm³), ce qui permet d'absorber plus efficacement les rayonnements que les tissus biologiques environnants, malgré un numéro atomique équivalent. De plus, les nanodiamants ont une très bonne stabilité colloïdale, même sans fonctionnalisation qui atténuerait le transfert des électrons générés à leur environnement. Enfin, l'excellente conductivité thermique du diamant assure une libération très efficace de la chaleur résultant de l'irradiation (par exemple par un rayonnement ionisant) à la surface des nanodiamants. Les nanodiamants permettent donc de générer efficacement des radicaux libres dans les cellules tumorales, et/ou de la chaleur, tout en utilisant un matériau à base de carbone peu coûteux, biocompatible et stable en milieu biologique sans fonctionnalisation nécessaire.

La présente invention porte donc, en premier lieu, sur l'utilisation d'un nanodiamant, comme médicament, en combinaison avec un rayonnement ionisant, pour générer localement des radicaux libres et détruire des cellules cibles. Selon une mise en oeuvre particulière de l'invention, une production de chaleur est couplée à la production de radicaux libres. L'utilisation de nanodiamant comme médicament et/ou comme outil de diagnostic générant des radicaux libres découle des propriétés mises en évidence par les inventeurs et décrites ci-dessus.

Dans le cadre de la présente invention, l'action thérapeutique des nanodiamants est le fait des dommages causés, directement ou indirectement, par les radicaux libres généré à la surface des nanodiamants, aux molécules situées aux alentours immédiats des nanoparticules, et en particulier à moins de 10 nm de la surface des nanoparticules. Dans les applications visant la destruction de cellules, il est donc préférable que les nanoparticules pénètrent dans les cellules, pour que les radicaux libres causent des dommages aux acides nucléiques cellulaires (ADN et ARN nucléaires et/ou ARNs cytoplasmique et/ou ADN mitochondrial, *etc*.). Pour cela, des nanodiamants dont le diamètre moyen est inférieur à 100 nm, voire inférieur à 50 nm ou même inférieur à 10 nm, sont préférés pour mettre en oeuvre l'invention.

Les inventeurs ont observé que des nanodiamants ayant subi un traitement de surface particulier pour créer des fonctions CHₓ (x = 1, 2 ou 3) et/ou du carbone sous forme graphitique en surface (par plasma hydrogène, recuits à haute température sous vide ou sous hydrogène par exemple) sont particulièrement efficaces. L'expression "nanodiamant graphitisé" désigne ici tout nanodiamant contenant, en surface, des atomes de carbone hybridé sp², Bien entendu, la présence d'atomes de carbone hybridé sp² en surface du nanodiamant n'exclut pas la présence d'autres groupements chimiques à la surface de ces nanodiamants. De même, l'expression "nanodiamant hydrogéné" désigne tout nanodiamant contenant des fonctions de type C-H, CH₂ ou CH₃ en surface. Les nanodiamants dont la surface a été (partiellement ou totalement) graphitisée et/ou hydrogénée, quand ils sont exposés à l'air ou dispersés en milieu aqueux, présentent des propriétés de conduction de surface qui leur donnent, une affinité électronique négative. En parallèle, leur surface devient favorable à l'adsorption préférentielle des molécules étant à l'origine des radicaux libres en milieu biologique (H₂O, O₂ et NO₂ principalement), permettant ainsi un transfert d'électrons du nanodiamant vers ces molécules, qui s'effectue spontanément. Sous rayonnement ionisant, ce transfert d'électrons est amplifié et crée des radicaux libres de manière particulièrement efficace. En outre, l'adsorption préférentielle de molécules comme H₂O, O₂ et NO₂ à la surface de ces nanodiamants modifiés permet, en apportant ces molécules dans les cellules, de surmonter une des causes principales de la radiorésistance des cellules tumorales, qui est le manque d'oxygène dans ces cellules (hypoxie), limitant la génération de radicaux libres oxygénés.

Les nanodiamant avant des fonctions CHx et/ou du carbone sous forme graphitique en surface ont de plus une permittivité diélectrique très importante, qui permet de les utiliser comme source de chaleur sous un champ électromagnétique oscillant à des fréquences entre 100 et 10⁶ Hz (Batsanov *et al,* 2012). Ces nanodiamants possèdent aussi des propriétés d'absorption des rayonnements infrarouges, comme les autres nanomatériaux carbonés, induisant aussi une libération de chaleur (Yang *et al,* 2010). Pour obtenir une production de chaleur par les nanodiamants, des infrarouges de longueur d'onde comprise entre 600 et 1400 nm seront préférentiellement utilisés, cette gamme correspondant aux infrarouges absorbés par les tissus biologiques. Les propriétés de génération de radicaux libres peuvent alors être couplées à de l'hyperihermie.

Selon une mise en oeuvre préférée, la présente invention porte donc sur un nanodiamant dont la surface a été au moins partiellement graphitisée et/ou hydrogénée, pour utilisation pour générer des radicaux libres à visée thérapeutique et/ou diagnostique, éventuellement couplés à de la chaleur. En particulier, la présente invention porte sur un nanodiamant pour utilisation en combinaison avec un rayonnement, comme médicament et/ou comme outil de diagnostic générant des radicaux libres.

Parmi les rayonnements susceptibles de provoquer la génération de radicaux libres à la surface des particules de nanodiamant, on peut citer les rayonnements ionisants, parmi lesquels des ondes électromagnétiques telles que les rayons X, les rayons gamma et les ultra-violets, ainsi que des rayonnements l'articulaires, notamment un rayonnement constitué de protons, de hadrons ou de particules béta (B⁺ ou β⁻). Selon un mode de réalisation préféré, la présente invention porte donc sur un nanodiamant tel que ceux décrits plus haut, pour utilisation en combinaison avec un rayonnement ionisant, comme médicament et/ou comme outil de diagnostique.

L'homme du métier adaptera les paramètres du rayonnement utilisé (nature, énergie, puissance et durée d'irradiation) en fonction de la profondeur du tissu à atteindre et d'autres paramètres cliniques et techniques. A titre purement indicatif, certaines valeurs sont présentées dans le tableau 1 ci-dessous.

Toutefois, d'autres rayonnements peuvent être utilisés, à la place ou en combinaison avec les rayonnements cités ci-dessus. Parmi ces rayonnements de natures diverses, on peut citer les rayonnements visible, infrarouge, micro-ondes, neutrons, notamment pour obtenir une production de chaleur. Les irradiations peuvent être multiples, tant par la nature des rayonnements utilisés que par l'application des rayonnements en plusieurs fois.

**Tableau 1**

| Nature du rayonnement | Energie | durée | Pénétration | Organes |
|---|---|---|---|---|
| UV | 3-120 eV | Quelques minutes | Superficielle | Peau et cavités (exemple : vessie) |
| Rayons X | 20 à 150 keV | Quelques minutes | Quelques millimètres | Peau et cavités (exemple : vessie) |
| Rayons X (ortho voltage) | 200 à 500 keV | Quelques minutes | Jusqu'à 6 cm | Organes superficiels, muscles |
| Rayons X (méga voltage) | 1000keV à 25000keV | Quelques minutes | Tout le corps | Organes profonds (exemple : prostate) |

Selon une mise en oeuvre particulière de l'invention, au moins une partie des radicaux libres générés sont des radicaux libres oxygénés. Les inventeurs ont également montré que l'azote pouvait s'adsorber à la surface des nanodiamants. Aussi, selon une mise en oeuvre de l'invention, au moins une partie des radicaux libres générés sont des radicaux libres azotés.

Les radicaux libres provoquant des ruptures de l'ADN. les nanodiamants décrits plus haut trouvent une application avantageuse dans la destruction de cellules cibles telles que des cellules cancéreuses. Selon un mode de réalisation préféré, la présente invention concerne donc l'utilisation d'un nanodiamant tel que décrit ci-dessus pour traiter une tumeur solide, ladite utilisation étant basée sur la propriété intrinsèque du nanodiamant de générer des radicaux libres sous irradiation.

Selon une mise en oeuvre particulière de l'invention, les particules de nanodiamant sont fonctionnalisées. En particulier, un nanodiamant selon l'invention peut être lié à une molécule de ciblage, par liaison covalente ou non. Plusieurs techniques de greffage de molécules à la surface de nanodiamants ont été décrites dans l'art antérieur, et l'homme du métier est en mesure de choisir, en fonction notamment du type de molécule de ciblage, celle qui lui convient le mieux. A titre d'exemples non limitatifs de méthodes de greffage de molécules de ciblage à la surface de nanodiamants, on peut citer :
- un couplage type peptidique *via* la formation d'une fonction amide ou ester entre le nanodiamant et la molécule de ciblage (Huang et Chang, 2004)
- un couplage direct de la molécule de ciblage sur le nanodiamant par la création d'une liaison C-C entre les deux entités (Girard *et al.,* 2011)
- un couplage de type cycloaddition via la présence de carbones à l'état d'hybridation sp² ou sp sur le nanodiamant et/ou sur la molécule de ciblage (Jarre *et al.,* 2011)
- un couplage de type silanisation via la présence de fonction silanes sur la surface du nanodiamant et/ou sur la molécule de ciblage (Krüger *et al.,* 2006)
- un couplage non covalent, basé sur des interactions électrostatiques et/ou la formation de liaisons hydrogène entre le nanodiamant et la molécule cible (Chen *et al.,* 2010).

Ces couplages peuvent s'effectuer soit directement à la surface des nanodiamants, soit par l'intermédiaire de molécules préalablement greffées ou adsorbées à la surface des nanodiamants. Pour les nanodiamants hydrogénés, le couplage direct par la création d'une liaison C-C est le plus efficace. Pour les nanodiamants graphitisés, le couplage par cydoaddition est le plus approprié.

Selon un mode de réalisation préféré des nanodiamants ciblés selon l'invention, le ciblage est assuré par au moins un ligand biologique reconnu par un récepteur surexprimé à la surface de certaines cellules. Les ligands biologiques permettant de cibler spécifiquement certaines cellules peuvent être :
- des peptides, par exemple le peptide RGD, ou leurs dérivés ou leurs analogues (ex : le peptide octéotrate, analogue de la somatostatine, un analogue de la bombésine, de la neurotensine, l'EGF. le VIP...),
- des protéines, des anticorps, ou leurs dérivés ou leurs analogues,
- des sucres, notamment des monosaccharides (ex : glucose, galactose, glucosamine ou galactosamine), des oligosaccharides, des polysaccharides, ou leurs dérivés ou leurs analogues,
- des oligonucléotides, ADN, ARN, leurs dérivés ou leurs analogues,
- des molécules organiques (telles que le folate ou le pamidronate biphosphonaté),
- des complexes organométalliques.

Leur activité de ciblage est due à la reconnaissance moléculaire de ces ligands par des récepteurs surexprimés à la surface des cellules de la zone d'intérêt.

Des ligands particulièrement préférés pour mettre en oeuvre l'invention sont des ligands de molécules fréquemment surexprimées à la surface des cellules tumorales. Par exemple des peptides comportant le motif RGD, tels que le cyclo(RGDfK), le cyclo(RGDyK) ou le cyclo(RGDfV), peuvent avantageusement être utilisées. Ces peptides reconnaissent l'intégrine αᵥβ₃, qui est surexprimée à la surface des cellules tumorales et des cellules endothéliales lors de la néoangiogénèse tumorale. L'utilisation de ces ligands dans les nanodiamants selon l'invention permet donc de cibler les tumeurs et leur vascularisation, pour les détruire par la production de radicaux libres, éventuellement couplée à la production de chaleur. Un autre ligand préféré est par exemple un peptide comportant le motif NGR décrit par Curnis *et al.* (2002), lequel cible également les néo-vaisseaux.

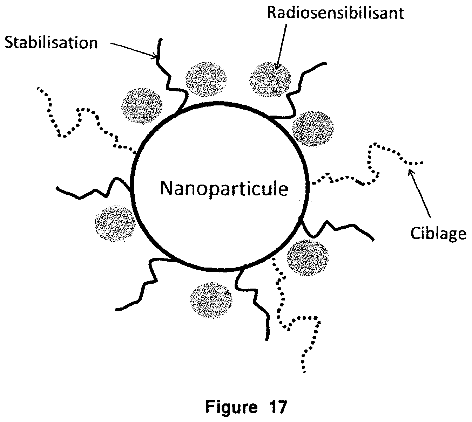

Bien entendu, d'autres molécules peuvent êtres greffées à la surface des nanodiamants utilisés dans le cadre de la présente invention, par exemple pour augmenter leur stabilité en milieu biologique, ou pour en effectuer un marquage (fluorophore, marqueur radioactif, *etc.*) (Figure 17).

De manière avantageuse, les nanodiamants selon la présente invention peuvent être suivis par des méthodes d'imagerie utilisant la luminescence intrinsèques des centres colorés dans les nanodiamants (centres N-V ou autre) (Chang *et al.,* 2008). Des méthodes de spectroscopies peuvent aussi être utilisées, notamment la spectroscopie Raman (Chao *et al.,* 2007). Le praticien peut donc vérifier la présence des nanodiamants au niveau de la zone ciblée, par exemple au niveau d'une tumeur solide, avant de soumettre cette zone à l'irradiation qui entraînera la génération de radicaux libres. Ceci permet de diminuer encore les effets secondaires du traitement.

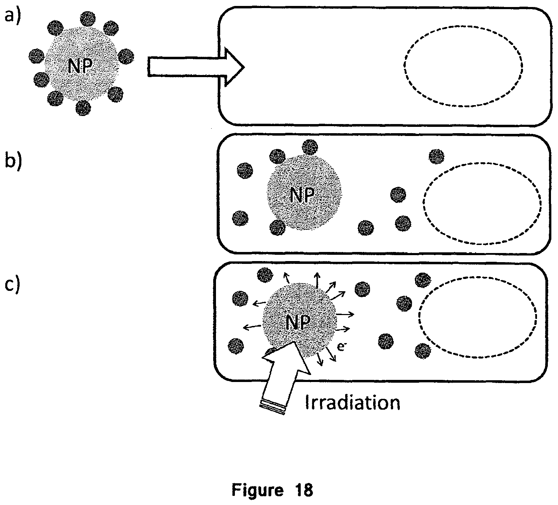

Comme mentionné plus haut, les nanodiamants sont connus pour leur capacité à vectoriser des molécules. Selon un mode de réalisation particulier, la présente invention propose d'utiliser des nanodiamants à la fois pour leurs propriétés de vectorisation (fonction passive) et pour leurs propriétés de génération de radicaux libres sous irradiation (fonction active). Le principe de fonctionnement de cet aspect de l'invention est illustré à la Figure 18. Le couplage des propriétés de vectorisation des nanodiamants à ses propriétés de génération de radicaux libres est particulièrement avantageux dans le cadre de complexes muitimodaux nanodiamants/radiosensibilisants, En effet, les radiosensibilisants permettent une réponse sélective en attaquant des voies biologiques spécifiques aux cellules tumorales tandis que les nanodiamants augmentent la quantité de radicaux libres générés. L'association de ces deux composantes permet d'augmenter significativement l'efficacité et la sélectivité par rapport à l'utilisation de manière isolée de nanoparticules ou de molécules radiosensibilisantes : d'une part, l'utilisation de nanodiamants permet de transporter les radiosensibilisants dans les cellules tumorales de manière ciblée et de les libérer progressivement dans ces cellules, si bien que la biodisponibilité des radiosensibilisants est améliorée et, d'autre part, les radiosensibilisants vont inactiver les défenses des cellules tumorales, donc les radicaux générés par les nanodiamants vont provoquer davantage de ruptures d'ADN dans les cellules tumorales. Ce couplage permet *a priori* d'augmenter les concentrations de radiosensibilisants délivrés dans les cellules tumorales et de réduire la dose de radiation nécessaire pour éliminer ces cellules.

Selon cette approche, la présente invention concerne une composition comprenant un nanodiamant tel que décrit ci-dessus, fonctionnalisé ou non par une molécule de ciblage, ainsi qu'une molécule radiosensibilisante. Cette molécule radiosensibilisante peut être liée audit nanodiamant par liaison covalente, ou simplement adsorbée à sa surface.

Différents types de molécules radiosensibilisantes, aussi appelés "radiosensibilisants", ont été décrits et peuvent être utilisés pour mettre en oeuvre la présente invention. A ce titre, on peut notamment citer des radiosensibilisants de type chimique, dont une liste non exhaustive comprend le misonidazole, le metronidazole, l'etanidazole, le pimonidazole, les phenylpropanoides et l'acide monoiodoacétique. Ces molécules peuvent être fixées sur les nanodiamants de façon covalente, éventuellement via des bras clivables en milieu cellulaire, permettant la libération de ces molécules après l'internalisation du nanodiamant. A titre d'exemple de bras clivables, on peut citer un polymère PEG/poly-lysine, un bras peptidique clivé par une enzyme surexprimée dans le type de tumeur ciblé (par exemple la MMP2 ou la cathepsine D), ou encore un bras comprenant un pont disulfure, lequel sera clivé par les thiorédoxines dans les lysosomes et endosomes des cellules. Alternativement, le radiosensibilisant chimique peut être lié au nanodiamant par des liaisons faibles (adsorption électrostatique soit directe, soit par l'intermédiaire d'un polymère chargé).

Comme évoqué plus haut, un nouvel axe de recherche dans le domaine de la radiosensibilisation concerne l'utilisation d'acides nucléiques capables de moduler l'expression de certaines protéines impliquées dans la réparation de l'ADN. La présente invention porte donc également sur une composition comprenant un nanodiamant tel que décrit plus haut et une molécule radiosensibilisante constituée d'une molécule d'acide nucléique adsorbée à la surface du nanodiamant. Les acides nucléiques préférés pour mettre en oeuvre cet aspect de l'invention sont des ARN interférents capables d'inhiber l'expression d'un gène responsable de la réparation de l'ADN, comme par exemple un ARN interférent inhibant l'expression du gène POLQ.

Bien entendu, les compositions de l'invention seront avantageusement utilisées en combinaison avec un rayonnement, notamment comme médicament anticancéreux, et de préférence dans des conditions telles que le rayonnement, induit la production, à la surface des nanodiamants, de radicaux libres et/ou de chaleur. Le terme "en combinaison" indique que l'effet recherché est obtenu lorsque les cellules, tissus ou organes d'intérêt, ayant incorporé en partie des nanoparticules de l'invention, sont excités par le rayonnement. Toutefois, il n'est pas nécessaire que les particules et les rayons soient administrés simultanément, ni selon le même protocole.

Un autre objet particulier de l'invention réside dans une méthode pour induire ou causer la lyse ou la destruction de cellules cancéreuses, *in vitro, ex vivo* ou *in vivo,* comprenant la mise en contact de cellules cancéreuses avec un ou des nanodiamants ou compositions tels que décrits précédemment, pendant une période de temps suffisante pour leur permettre de pénétrer dans les cellules cancéreuses et, le cas échéant, pour permettre la libération des radiosensibilisants, suivie de l'exposition des cellules à des rayons tels que définis ci-dessus, ladite exposition induisant la génération de radicaux libres et/ou de chaleur à la surface des nanodiamants et provoquant la lyse ou la mort desdites cellules. Le cas échéant, une étape intermédiaire de détection des nanodiamants, par exemple par spectroscopie Raman, est effectuée avant l'étape d'exposition aux rayons, afin de contrôler la localisation des nanodiamants.

L'invention concerne également une méthode de traitement du cancer, comprenant l'administration à un patient atteint d'un cancer d'une composition comprenant des nanodiamants tels que définis précédemment, de préférence fonctionnalisés par une molécule de ciblage, dans des conditions permettant aux nanodiamants ou agrégats nanoparticulaires de pénétrer dans les cellules cancéreuses, et le traitement ultérieur du patient en présence d'une source d'excitation, par exemple choisie parmi les rayons X, les rayons gamma, les UV, les protons et les hadrons, conduisant à une altération, une perturbation ou une destruction fonctionnelle de cellules cancéreuses du patient, traitant ainsi le cancer.

Le terme "traitement" désigne ici toute amélioration des signes cliniques, comme notamment une diminution de la taille ou du développement d'une tumeur ou d'une zone tissulaire pathologique, la suppression ou la destruction de cellules ou tissus pathologiques, un ralentissement de la progression de la pathologie, une réduction de la formation de métastases, une régression ou une rémission complète, *etc*. Les nanodiamants et compositions de l'invention peuvent également être utilisées *in vitro* ou *ex vivo.*

L'invention est utilisable pour traiter tout type de cancer, notamment les tumeurs solides, métastasées ou non, par exemple choisies parmi les cancers du poumon, foie, rein, vessie, sein, tête-et-cou, cerveau, ovaires, prostate, peau, intestin, colon, *etc.* Les rayons peuvent être appliqués à tout moment après l'administration des particules, en une ou plusieurs fois, en utilisant tout système de radiothérapie ou de radiographie déjà disponible. Les nanodiamants et compositions de l'invention peuvent être administrés par différentes voies, de préférence par injection, systémique ou locale, ou de manière orale. Des injections ou administrations répétées peuvent être envisagées, si nécessaire.

Les exemples suivants et les figures annexées illustrent l'invention sans toutefois limiter son étendue.

### Légendes des figures

**Figure 1** **:** Schéma du montage d'hydrogénation des nanodiamants (NDs) par plasma d'hydrogène (H₂) assisté par micro-ondes.
**Figure 2** **:** Clichés par Microscopie Electronique en Transmission Haute Résolution (METHR) de NDs initiaux (a), NDs après 1h (b) et 8h (c) de recuit sous vide à 750°C. Les plans diamants (111) et graphitiques (001) sont indiqués par les lignes blanches et grises, respectivement. Les reconstructions de surface graphitiques sont indiquées par des flèches blanches. La barre d'échelle est de 5 nm.
**Figure 3** **:** Spectres de Spectroscopie de Photoélectrons X (XPS) du niveau de coeur du carbone (Cls) des NDs initiaux (ND-initial), NDs après 1h (ND-1h), 5h (ND-5h) et 8h (ND-8h) de recuit sous vide à 750°C,
**Figure 4** **:** Evolution du potentiel Zeta des NDs-1h, ND-5h, ND-8h et NDs hydrogénés (ND-H) dans l'eau ultrapure en fonction du pH.
**Figure 5** **:** Distribution en taille des protéines du milieu DMEM + 10% de sérum de veau foetal (SVF) (noir), des NDs-5h dans l'eau déionisée (bleu), dans le MEM juste après ajout (vert) et après 5h (rouge). Les mesures sont effectuées à 37°C.
**Figure 6** **:** Cliché METHR de nanodiamants hydrogénés (a) et graphitisés en surface (b). Les plans (111) du diamant sont surlignés en plan et les reconstructions graphitiques de surface sont indiquées par les flèches blanches. La barre d'échelle est de 5 nm.
**Figure 7****:** Spectres XPS du niveau de coeur du carbone (Cls) de nanodiamants hydrogénés après dispersion dans l'eau (a) et après une heure de recuit sous vide à 400°C (b).
**Figure 8** **:** Spectres de Spectroscopie infrarouge à Transformée de Fourier (FTIR) de NDs initiales (ND initial), NDs-G après 1h (ND-1h) et 8h (ND-8h) de recuit sous vide.
**Figure 9** **:** Réponse cellulaire de la lignée Caki-1 après exposition aux NDs-COOH sans irradiation.
**Figure 10** **:** Réponse cellulaire de la lignée Caki-1 après exposition aux NDs-COOH après une irradiation de 4 Gy.
**Figure 11** **:** Réponse cellulaire de la lignée Caki-1 après exposition aux NDs-H sans irradiation.
**Figure 12** **:** Réponse cellulaire de la lignée Caki-1 après exposition aux NDs-H après une irradiation de 4 Gy.
**Figure 13** **:** Evolution des cellules Caki-1 après exposition aux NDs-COOH et NDs-H , sans irradiation.
**Figure 14** **:** Evolution des cellules Caki-1 après exposition aux NDs-COOH et NDs-H, après une irradiation de 4 Gy.
**Figure 15** **:** Stress oxydant induit par les NDs-H avec ou sans irradiation. Le témoin sans sonde illustre le fond de luminescence n'étant pas lié aux radicaux libres. L'autre témoin est exposé à la sonde fluorescente mais pas aux NDs-H. Les barres grises représentent l'intensité du stress oxydant dans les cellules non irradiées, et les barres blanches l'intensité du stress oxydant dans les cellules ayant subi un rayonnement de 4 Gy, une heure après cette irradiation.
**Figure 16** **:** Internalisation d'une sonde d'acide nucléique peptidique marquée avec le fluorophore Cy3 (ANP-Cy3) adsorbée à la surface de ND-H. Les observations ont été réalisées après 24h (A) ou 72h (B) d'incubation de cellules Caki en présence d'un mélange ND-H/ANP-Cy3. Les noyaux cellulaires ont été marqués au bisbenzimide Hoechst 33342.
**Figure 17** **:** Complexe nanoparticule de diamant / radiosensibilisant.
**Figure 18** **:** Principe de fonctionnement. a) incorporation du complexe nanodiamants (NP)/radiosensibilisants dans une cellule, b) libération des radiosensibilisants et inhibition des défenses de la cellule, c) génération d'électrons et formation de radicaux libres sous irradiation.

### EXEMPLES

### Exemple 1 : Production de nanodiamants (NDs) avant des reconstructions de surface graphitiques ou des fonctions hydrogénées en surface

Les NDs ayant des propriétés utiles pour la radiosensibilisation de cellules tumorales ont été modifiés par des traitements particuliers permettant la formation de reconstructions graphitiques (graphitisation) ou de fonctions hydrogénées de type CHₓ avec x=1, 2 ou 3 (hydrogénation). Des NDs comportant une combinaison de ces deux types de terminaisons de surface peuvent également être utilisés dans le cadre de la présente invention. Les méthodes décrites ci-dessous sont celles utilisées par les inventeurs pour l'hydrogénation (par plasma d'hydrogène assisté par micro-ondes) et la graphitisation (par recuit sous vide à haute température ou par exposition aux micro-ondes sous vide de NDs hydrogénés), mais ces terminaisons de surface particulières peuvent *a priori* être obtenues également par d'autres méthodes. Il est à noter que les conditions exposées sont à adapter en fonction de la chimie de surface initiale des NDs, qui peut varier d'un fournisseur de nanodiamants à l'autre. Les traitements décrits ici ont été optimisés pour des NDs de détonation produits par le Nanocarbon Institute au Japon (Prof. Eiji Osawa).

### 1.1. Hydrogénation par plasma hydrogène assisté par micro-ondes

### 1.1.1. Mode opératoire

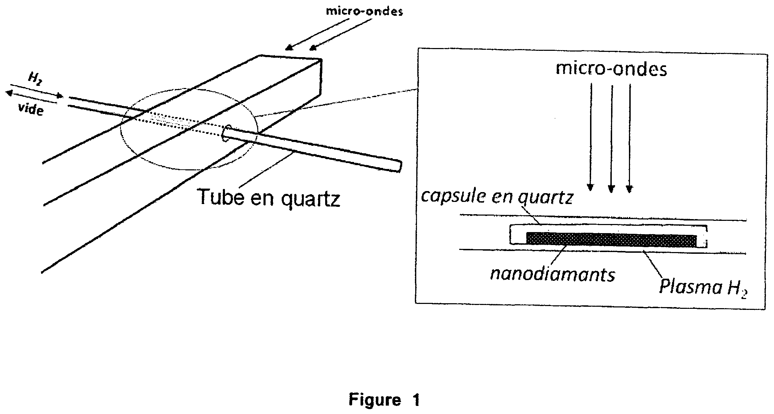

La méthode utilisée pour conférer des terminaisons hydrogénées aux NDs est décrite dans la référence de Girard *et al.,* 2010. Les NDs (environ 50-100 mg) sont introduits en voie sèche dans une cartouche en quartz, ou bien directement dans un tube en quartz, qui est inséré de façon perpendiculaire dans un guide d'onde connecté à un générateur de micro-ondes à 2.45 GHz (Sairem), comme présenté sur la figure 1. Le guide d'onde est refroidi à l'eau et le tube est refroidi à l'air comprimé. Ce tube est relié à un dispositif de pompage primaire et d'approvisionnement en hydrogène haute pureté N9.0 et argon gazeux.

Dans un premier temps, une série de purges sont réalisées via un pompage primaire dans le tube (pression < 0.1 mbar) et remise sous pression par de l'hydrogène haute pureté, puis l'hydrogène haute pureté est injecté jusqu'à atteindre une pression stabilisée à 12 mbar. Cette pression est soit maintenue tout au long du processus d'hydrogénation par isolation du tube (mode statique), soit maintenue par l'association d'un flux continu d'hydrogène et d'une vanne de régulation de pression sous consigne (mode dynamique). Une puissance micro-ondes de 300 W est utilisée pour induire la création d'un plasma dans le tube. La géométrie des micro-ondes dans le guide d'ondes est adaptée pour obtenir une puissance absorbée par le plasma maximale et une puissance réfléchie nulle au niveau du générateur. Le tube est régulièrement tourné et translaté manuellement afin de s'assurer que la majorité des NDs sont exposés au plasma. La durée normale d'exposition est de 20-30 min. Afin d'obtenir une hydrogénation complète, il est important d'effectuer une purge après 5 min de traitement afin d'évacuer des espèces oxydées désorbées de la surface des NDs : après arrêt des micro-ondes, le tube est pompé en vide primaire, puis de l'hydrogène pur est réintroduit dans le tube pour initier de nouveau la formation d'un plasma. Cette purge intermédiaire n'est pas utile dans le cas d'une hydrogénation sous flux dynamique d'hydrogène. A la fin du traitement, le tube est refroidi sous hydrogène jusqu'à ce qu'il soit à la température ambiante, puis le gaz résiduel est pompé. Le tube est remis à pression ambiante par introduction d'argon, puis les NDs peuvent être récupérés.

### 1.1.2. Caractérisation

Des caractérisations détaillées des propriétés de surface des NDs hydrogénés préparés de cette manière ont été publiées (Girard *et al.,* 2010; Girard *et al.,* 2011; Arnault *et al.,* 2011). La chimie de surface y est étudiée par spectroscopies d'électrons (XPS), infrarouge (FTIR) et Raman. De plus, trois greffages dont la sélectivité sur films de diamant hydrogénés est connue ont été appliqués à ces nanodiamants hydrogénés ; une sélectivité équivalente vis-à-vis de la présence des terminaisons hydrogénées des nanodiamants a été démontrée. Ceci montre en particulier que ces NDs présentent des propriétés d'affinité électronique négative (Girard *et al,* 2011). Ces propriétés sont à l'origine de leur utilisation pour générer des radicaux libres dans l'eau (voir exemple 2).

### 1.2. Graphitisation de NDs hydrogénés par exposition micro-ondes

Les NDs hydrogénés selon le procédé décrit ci-dessus peuvent être graphitisés à la suite de leur hydrogénation, *in situ,* par une simple ré-exposition à des micro-ondes sous vide primaire. En effet, les inventeurs ont observé que les NDs hydrogénés ont la capacité d'absorber les micro-ondes sous vide. Ainsi, en adaptant la géométrie de la cavité micro-onde, la plupart de la puissance micro-onde (les inventeurs ont utilisé 300 W pour 100 mg de NDs) est absorbée par les NDs et est convertie en chaleur. Une exposition de quelques secondes est suffisante pour permettre une élévation très rapide de la température des NDs, induisant la formation de reconstructions graphitiques en surface, comme cela se produit dans un procédé de graphitisation classique par recuit à haute température (voir ci-dessous). Une exposition supérieure à 1 minute, en revanche, conduit à la formation de nanoparticules entièrement graphitiques où le coeur diamant a totalement disparu. Cette méthode peut être une alternative aux recuits sous vide à haute température dont le protocole expérimental est détaillé dans le paragraphe qui suit.

### 1.3. Graphitisation de surface des nanodiamants par recuit sous vide

### 1.3.1. Mode opératoire

La surface des nanodiamants peut être graphitisée par recuit sous vide à haute température (entre 700°C et 900°C) (Petit *et al.,* 2011). Ces recuits sous vide sont effectués dans une chambre à parois métalliques dédiée, équipée d'un élément chauffant en carbure de silicium permettant d'atteindre des températures supérieures à 1000°C et un système combiné de pompage primaire et turbomoléculaire permettant l'obtention d'un vide secondaire dans la chambre (de l'ordre de 10⁻⁷ mbar).

Entre 50 et 100 mg de NDs en voie sèche sont déposés dans un creuset en alumine muni d'un couvercle du même matériau, qui est alors placé sur l'élément chauffant à l'intérieur de la chambre. Durant le recuit, la température du creuset est mesurée par une caméra infrarouge (FLIR SC300) au préalable calibrée d'après l'émissivité du creuset tandis que la température de l'élément chauffant est estimée avec un thermocouple. La chambre est ensuite pompée à température ambiante jusqu'à l'obtention d'une pression inférieure à 5 × 10⁻⁷ mbar), puis la température de l'élément chauffant est progressivement augmentée jusqu'à 1000°C (correspondant à 750°C pour le creuset), en maintenant la pression dans la chambre inférieure à 5 × 10⁻⁶ mbar. Une fois la température stabilisée, le creuset est laissé à température constante pour une durée déterminée, puis la température de l'élément chauffant est progressivement réduite jusqu'à la température ambiante. Le creuset est ainsi refroidi sous vide. Une fois ramené à température ambiante, l'enceinte du réacteur est remise à pression atmosphérique sous air, permettant la sortie du creuset. Les NDs peuvent alors être récupérés pour être remis en suspension.

### 1.3.2. Caractérisations

Typiquement, un recuit à 750°C pendant une heure est suffisant pour obtenir la formation de reconstructions graphitiques en surface, mais des recuits plus longs peuvent être utilisés pour augmenter le taux de couverture de la surface par ces reconstructions graphitiques. Des températures supérieures à 900°C induisent une graphitisation du coeur diamant, la graphitisation limitée à la surface des NDs est donc difficilement contrôlable au-dessus de 900°C.

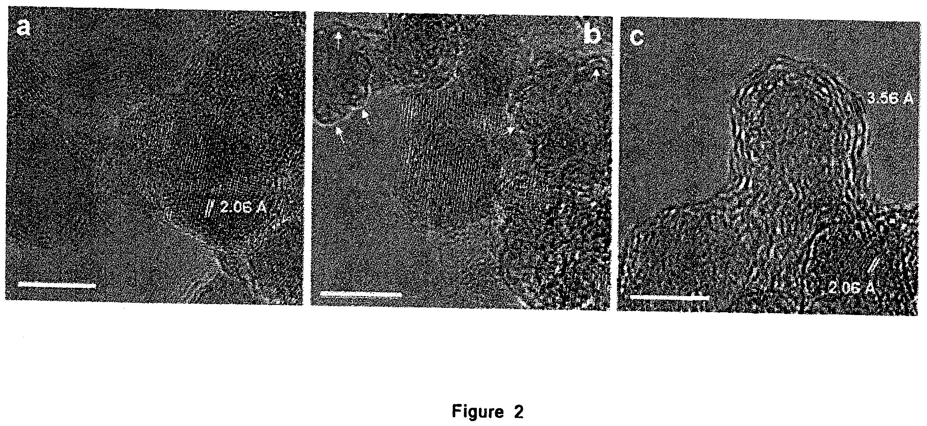

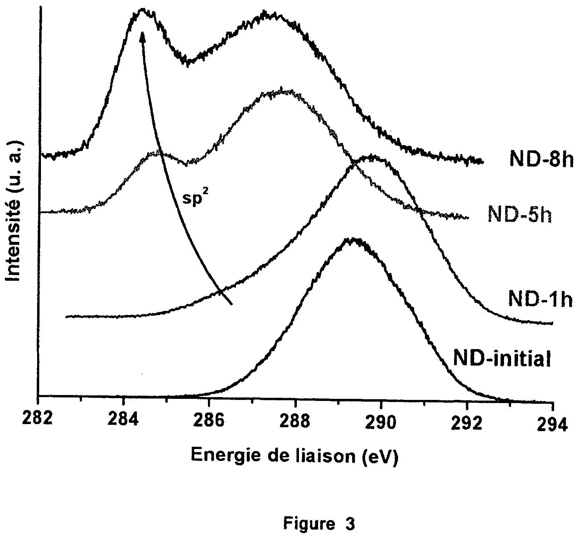

La graphitisation des NDs est validée par microscopie électronique en transmission haute résolution (METHR) après 1h et 8h de recuits sous vide à 750°C, correspondant à la température du creuset (Figure 2). Les clichés permettent d'observer les modifications de la structure atomique induites par les recuits. Cette graphitisation est aussi validée par l'analyse de la chimie de surface par spectroscopie de photo-électrons X (XPS). En effet, une composante liée au carbone hybridé sp² apparaît après recuit sous vide à basse énergie de liaison par rapport au carbone hybridé sp³ (Figure 3).

### Exemple 2 : Mise en suspension des NDs modifiés dans l'eau

Les NDs hydrogénés et/ou graphitisés sont ensuite mis en suspension colloïdale dans de l'eau ultrapure (18.2 MΩ.cm à 25°C) en utilisant une sonde plongeante de sonification (Hielscher UP400S) de 300 W fonctionnant à une fréquence de 24 kHz. Les NDs sont initialement introduits dans une solution d'eau ultrapure à une concentration de l'ordre de 5 à 10 mg/ml pour être ensuite exposés aux ultrasons pendant au minimum 2h. A la suite du procédé de sonification, et afin de séparer de la suspension les plus gros agrégats non dispersables, les suspensions sont centrifugées à 4800 tours/minute pendant 1h. Seul le surnageant est récupéré. Le diamètre hydrodynamique des NDs en suspension est mesuré par diffusion dynamique de la lumière (DDL) par un équipement dédié. La mesure du potentiel Zeta caractéristique de la charge de surface des nanodiamants en solution est effectuée sur le même équipement (Nanosizer ZS, Malvern) complété d'un module de titration automatique (MPT-2, Malvern) afin d'effectuer des mesures en fonction du pH.

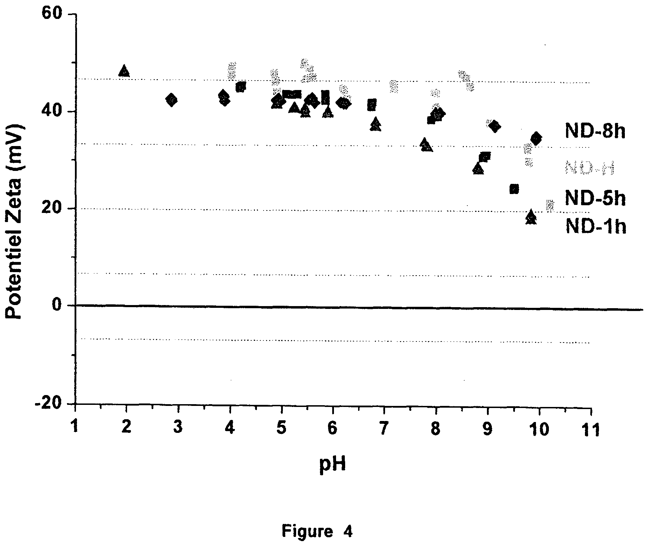

Les suspensions ainsi obtenues sont constituées d'agrégats de NDs dont le diamètre hydrodynamique est inférieur à 50 nm et présentent un potentiel Zeta positif dans l'eau ultrapure sur une large gamme de pH, comme indiqué pour des NDs recuits sous vide 1h (ND-1h), 5h (ND-5h) et 8h CND-8h) sur la figure 4. Une évolution similaire de la charge de surface est observée sur les NDs hydrogénés. En particulier, le potentiel Zeta élevé à pH physiologique permet d'assurer une bonne stabilité colloïdale des NDs modifiés dans cette gamme de pH.

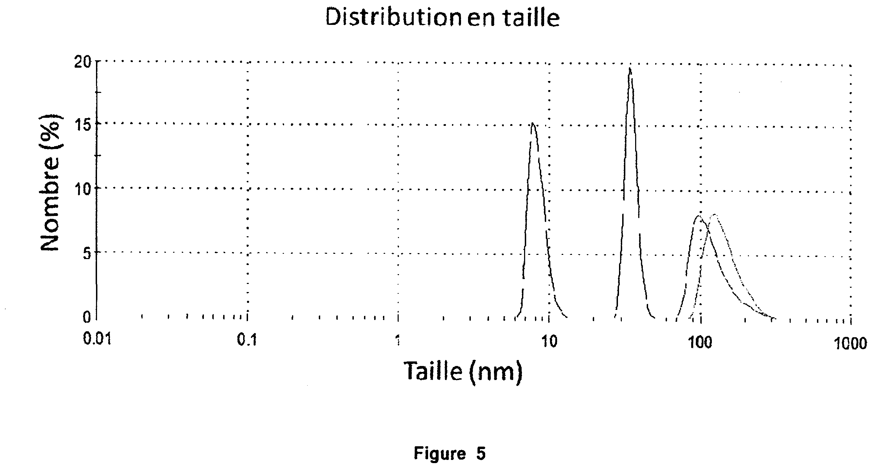

Ces NDs sont stables plusieurs mois dans l'eau mais aussi en milieu biologique comme l'illustre l'évolution du diamètre hydrodynamique des NDs-5h dans un milieu constitué de MEM (Minimal Essentiel Media) et de 10% de sérum de veau foetal, mesuré par DDL (Figure 5). Après plus de 6 mois dans l'eau, un diamètre moyenne de 35 nm est détecté pour les NDs-5h. Après ajout dans le milieu [MEM + sérum] à une concentration de 0.5 mg/ml environ, le diamètre augmente à 144 nm, ce qui est attribué à l'adsorption de protéines du sérum chargées négativement sur la surface positive des NDs. Après 5h d'incubation à 37°C, le diamètre est réduit à 121 nm, ce qui montre qu'il n'y a pas d'effet significatif d'agrégation au cours du temps en milieu biologique.

### Exemple 3 : Génération de radicaux libres à partir des nanodiamants (NDs) hydrogénés (NDs-H) /graphitisés (NDs-G)

L'effet d'amplification de la génération de radicaux libres à proximité des NDs-H/G est basé sur deux propriétés physiques : la forte densité d'atomes de carbone (de l'ordre de 10000 atomes pour un nanodiamant de 5 nm de diamètre) dans les NDs, permettant d'absorber efficacement les rayonnements, et leur capacité à transférer efficacement les électrons du coeur diamant à des espèces oxygénées fixées en périphérie des NDs.

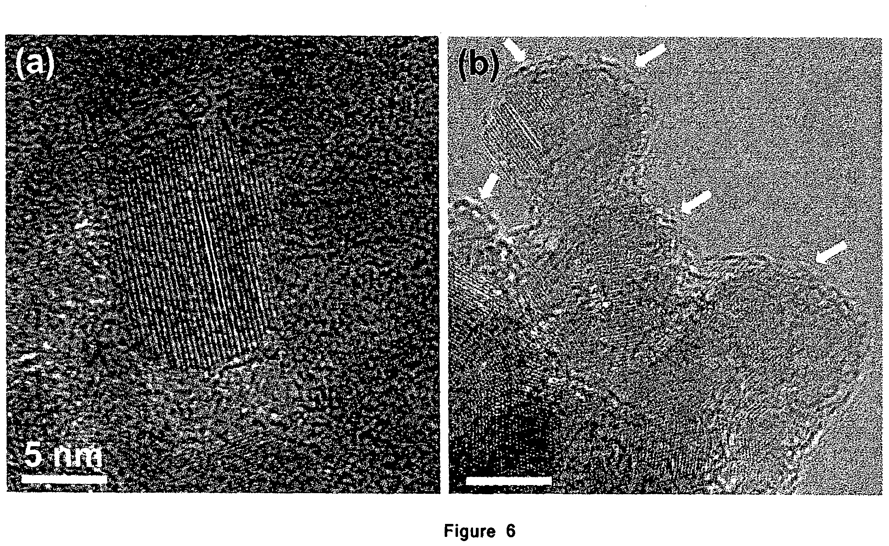

L'absorption des rayonnements ionisants est beaucoup plus importante dans les NDs que dans les tissus environnants à cause de la forte densité atomique du diamant (≈1.8x10²³ at.cm⁻³). En effet, la distance entre deux plans atomiques d'orientation (111) de la maille diamant est de 0.206 nm, comme l'illustre le cliché de microscopie électronique en transmission haute résolution (METHR) présenté sur la figure 6. Sous rayonnement, une forte concentration de photoélectrons et d'électrons secondaires sont créés et sont libérés localement à la surface des NDs. En effet, la surface des NDs-H et NDs-G se comporte respectivement comme la surface de films de diamant hydrogénés ou celle d'un plan de graphène. Ces deux surfaces sont connues pour permettre un transfert d'électrons très efficace vers des molécules adsorbées en surface (Chakrapani *et al.,* 2007; Ryu *et al.,* 2010).

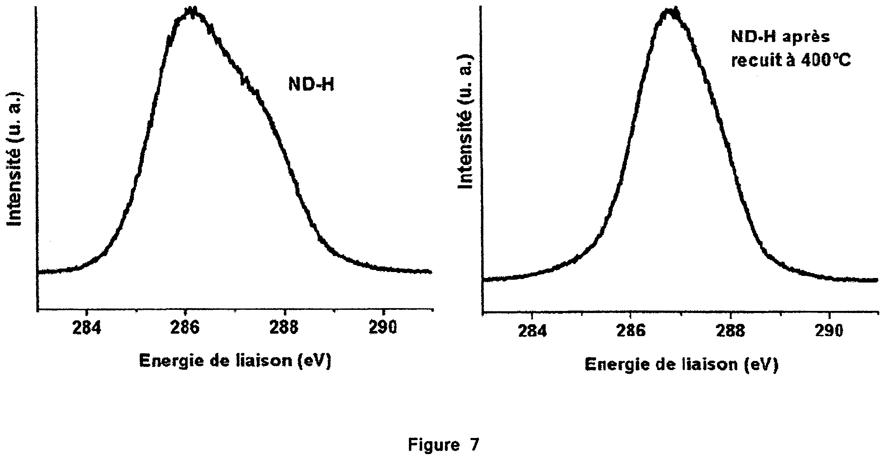

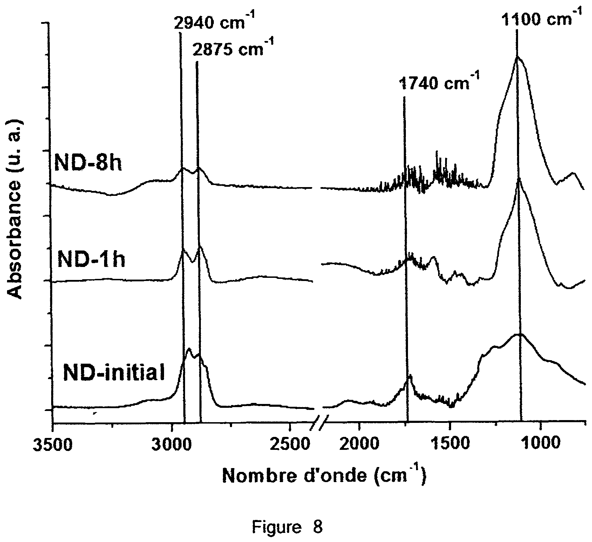

En parallèle, les NDs-H et NDs-G ont la possibilité d'adsorber efficacement des espèces oxygénées à leur surface. Ainsi, une forte concentration d'oxygène a été mesurée à la surface des NDs-H et NDs-G, représentant jusqu'à 6 % atomique d'après les spectres XPS, après dispersion dans l'eau ultra-pure. L'oxygène provient d'adsorption par des liaisons non covalentes de molécules d'eau (H₂O) et de dioxygène (O₂) ainsi que de liaisons covalentes C-O simple liaison pouvant être liées à des fonctions hydroxyles, éthers, époxydes ou endoperoxides. Cet oxygène, lié de manière covalente à la surface des NDs, est caractérisé par la présence d'un épaulement à haute énergie de liaison sur les spectres du niveau de coeur du carbone Cls par spectroscopie d'électrons X (XPS) présenté sur la figure 7a. En revanche, cet oxygène est faiblement lié puisque un recuit sous vide à 400°C permet de désorber la plupart de cet oxygène (Figure 7b). Par spectroscopie infrarouge (FTIR), après désorption des espèces adsorbées de manière non covalente par un recuit sous vide à 200°C, une importante bande à 1100 cm⁻¹ a été observée, qui peut être lié à des fonctions de type éthers, époxydes ou endoperoxides (Figure 8), validant les résultats obtenus par XPS.

Ainsi, les électrons générés par irradiation sont transférés à ces molécules adsorbées sur la surface des NDs. Ces molécules étant des précurseurs de radicaux libres oxygénés (O₂, HO , H₂O₂, ...), le transfert d'électrons venant des NDs induit une forte production de radicaux libres à la surface des NDs. Il est à noter que de l'azote a aussi été mesuré par XPS ; il est donc possible que des molécules azotées soient aussi adsorbées à la surface des NDs, impliquant la génération de radicaux libres azotés.

L'adsorption d'oxygène sur la surface induit un potentiel Zeta positif des NDs-H et NDs-G, assurant une bonne stabilité colloïdale par stabilisation électrostatique, même en milieu biologique. L'environnement biologique où se trouvent les NDs est donc directement exposé aux radicaux libres générés à la surface des NDs.

### Exemple 4 : Index cellulaire et stress oxydatif mesuré dans la lignée tumorale Caki-1, sous irradiation gamma en présence de NDs-COOH et NDs-H

L'effet radiosensibilisant des nanodiamants a été étudié sur une lignée tumorale du rein Caki-1, connu pour être particulièrement radiorésistante. Des cellules exposées à des NDs-COOH et NDs-H à 3 concentrations (10, 100 et 500 µg/ml), ainsi que des cellules sans NDs ont subi à un rayonnement de 4 Gray (Gy).

L'évolution de l'index cellulaire, caractéristique de réponse globale des cellules (morphologie, adhésion, viabilité, ...), a été suivi en temps réel sur 120h après irradiation par impédance-métrie en utilisant le système xCELLigence (Roche).

Le stress oxydatif a ensuite été évalué par observation des cellules par microscopie optique et quantifié par cytométrie en flux.

### 4.1. Résultats sur les NDs-COOH

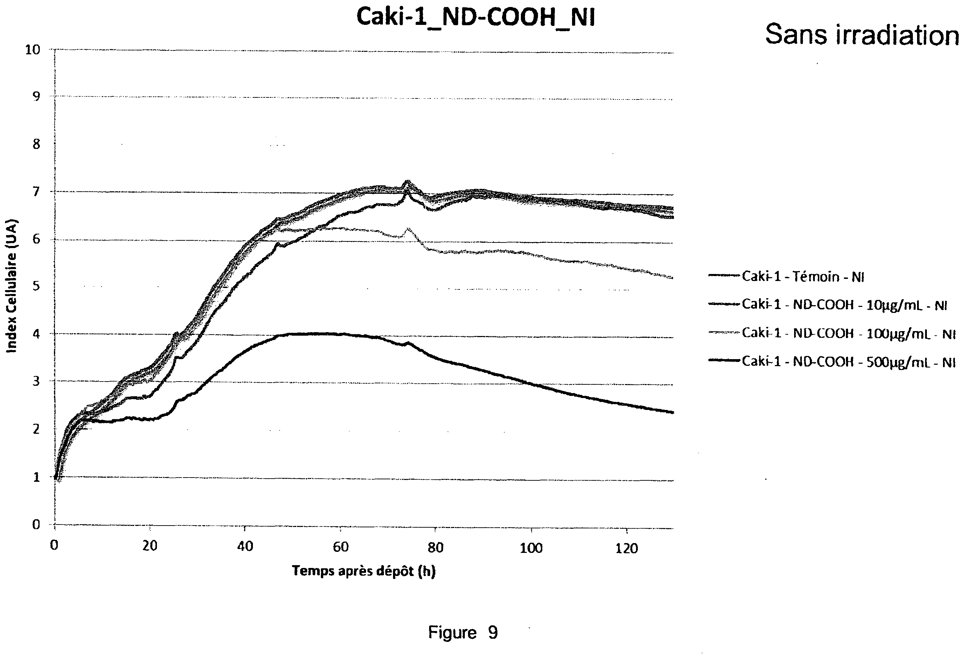

Après exposition aux NDs-COOH, l'index cellulaire évolue de manière équivalente jusqu'à 48h pour les concentrations de 10 et 100 µg/ml (Figure 9). Une très faible décroissance est observée à 100 µg/ml pour des temps plus longs. En revanche, l'index cellulaire est fortement diminué pour la concentration 500 µg/ml.

Ces résultats montrent que les NDs-COOH ne sont pas toxiques pour des concentrations inférieures à 100 µg/ml, mais qu'une certaine toxicité peut être observée à plus forte concentration. La toxicité dépend donc de la dose.

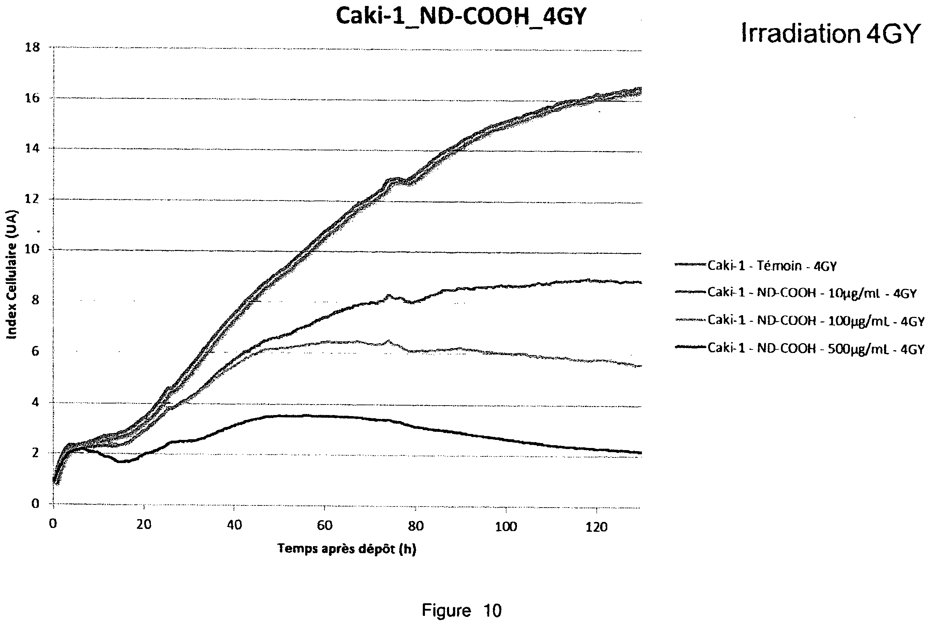

Après irradiation de 4 Gy, la croissance de l'index cellulaire du témoin montre que cette irradiation est trop faible pour créer une toxicité significative sans nanoparticules (Figure 10). En revanche, l'index cellulaire est divisé par 2 par rapport au témoin après une exposition aux NDs-COOH à une concentration de 10 µg/ml, voire plus pour les concentrations plus élevées.

Les NDs-COOH ont donc bien un effet radiosensibilisant, qui dépend de la dose de NDs-COOH injectée dans les cellules. De plus, ces NDs ne sont pas toxiques à des concentrations inférieures à 100 µg/ml.

### 4.2. Résultats sur les NDs-H

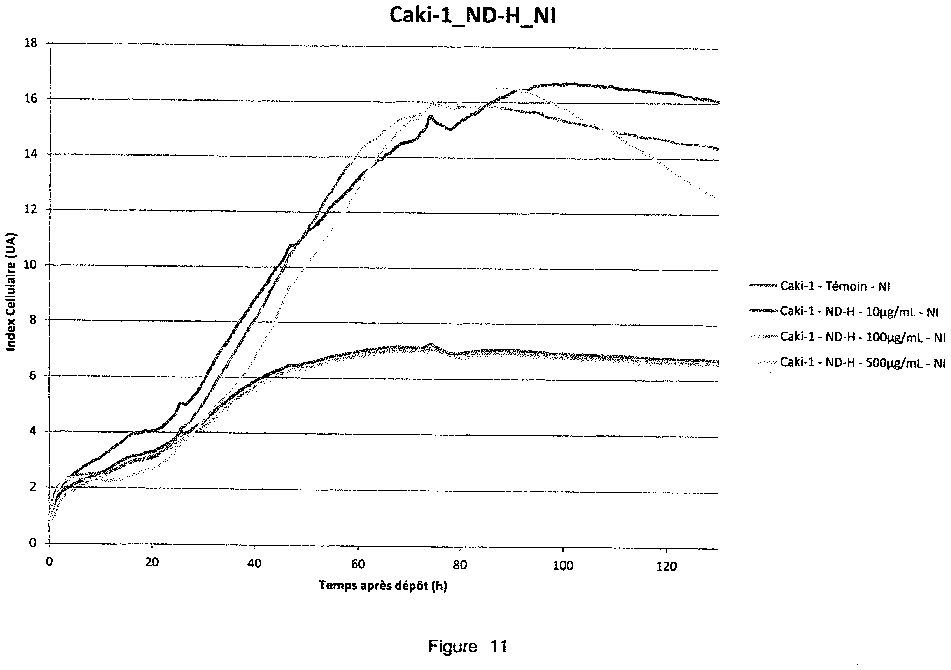

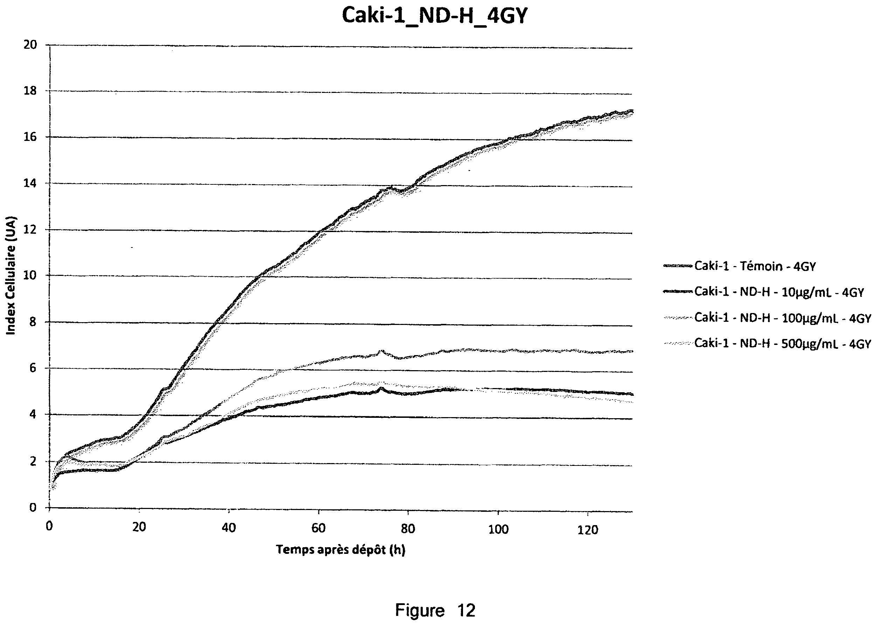

Le même protocole a été appliqué avec des NDs-H (Figures 11 et 12). Il est à noter que la toxicité des NDs-H est encore plus faible que les NDs-COOH puisqu'aucune toxicité n'est détectée même pour la concentration de 500 µg/ml, ce qui se serait traduit par une diminution de l'index cellulaire. La décroissance observée après 90h est probablement due à une saturation du signal détecté par impédance-métrice due à la forte concentration de NDs-H utilisés. On observe en revanche une augmentation significative de l'index cellulaire, qui peut résulter, par exemple, d'une augmentation de taille des cellules après incorporation des NDs-H.

Après irradiation, le témoin suit la même croissance que dans le cas précédent. En revanche, avec la présence de NDs-H. la toxicité est très importante. L'index cellulaire est ainsi divisé par 3,4 pour une concentration de 10 µg/ml. La toxicité ne semble pas dépendre de la dose de NDs-H puisque une évolution similaire de l'index cellulaire est observée à des concentrations plus élevées. Ce résultat est cohérent avec une toxicité induite par un stress oxydatif à très faible concentration en NDs-H, uniquement sous irradiation.

### 4.3. Résultats liés au stress oxydatif

Dans un premier temps, le stress oxydatif a été évalué en observant la morphologie des cellules par microscopie optique.

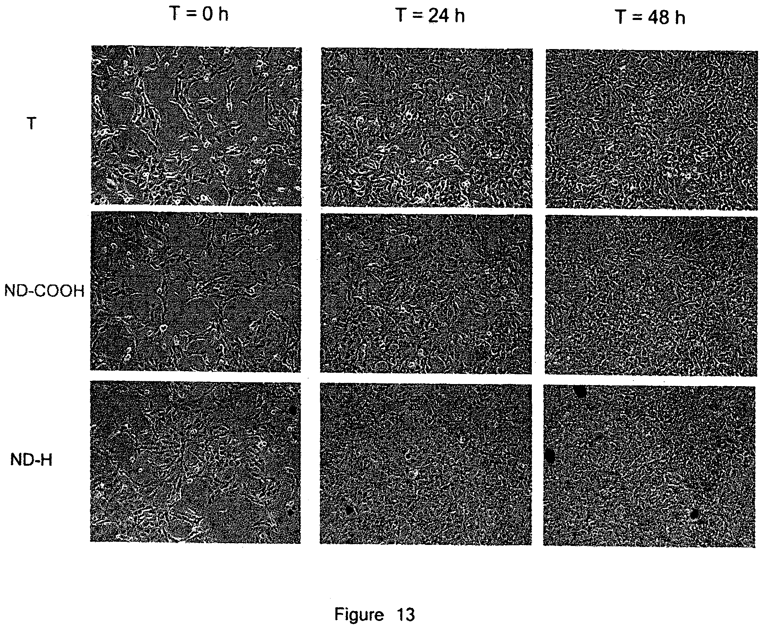

Il n'y a pas d'évolution particulière au niveau des cellules témoins sans/avec irradiation. Après ajout de nanodiamants, on observe la formation de vacuoles (apparaissant avec un contraste blanc), caractéristiques d'une toxicité induite par stress oxydatif (Figure 13). Le nombre de vacuoles augmente avec le temps.

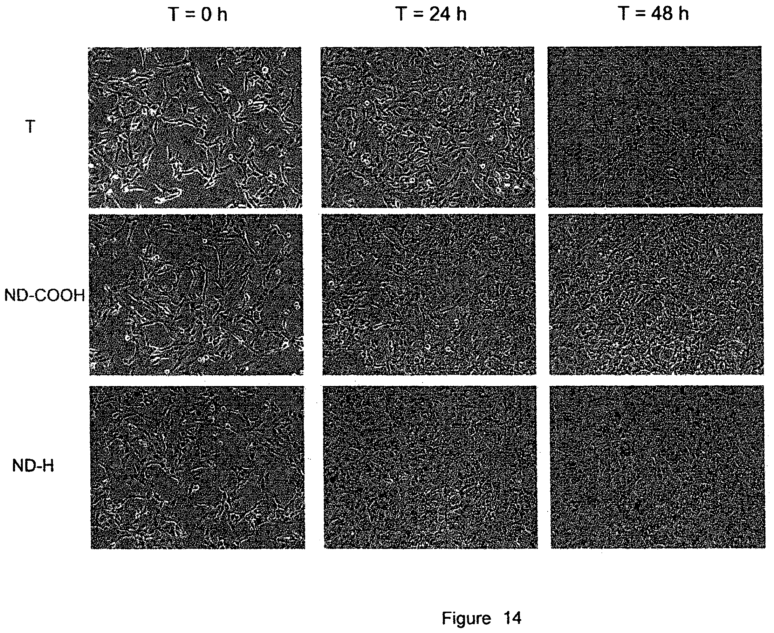

La concentration de vacuoles augmente significativement après irradiation dans les cellules exposées aux NDs (Figure 14), ce qui est en accord avec les résultats précédents.

Des mesures quantitatives du stress oxydatif ont été effectuées en mesurant la fluorescence d'une sonde sensible aux radicaux libres oxygénés (2',7'-dichlorofluorescein) par cytométrie en flux. Une fois exposées aux différentes conditions expérimentales (NDs-H, irradiation, NDs-H + irradiation), les cellules ont été détachées de leur support de culture, remises en suspension, puis incubées 10 minutes en présence de cette sonde. Une fois entrée dans les cellules, la sonde peut rester sous forme réduite non fluorescente ou s'oxyder et donc émettre un signal fluorescent. L'intensité de fluorescence est directement liée à la quantité de radicaux libres oxygénés, ce qui permet une quantification relative sur stress oxydatif (Chen *et al*., 2010). Cette méthode permet la mesure de radicaux libres oxygénés total intracellulaire, contrairement à la mesure de protéines oxydées par exemple.

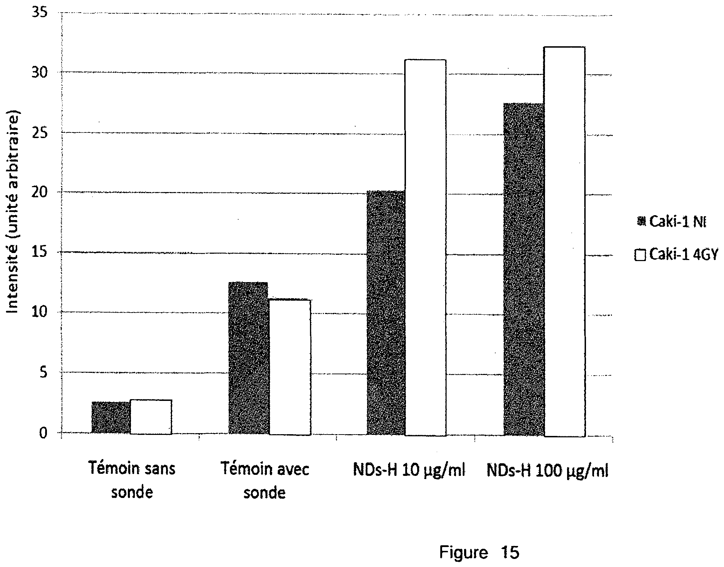

Les résultats obtenus sur la Figure 15 montrent que :
- Pour les cellules exposées aux NDs-H et non irradiées, le stress oxydant induit dépend de la concentration. Il est doublé pour une concentration en NDs-H de 100 µg/ml.
- Pour les cellules irradiées et non exposées aux NDs-H, le stress oxydant est, à 1h après irradiation, identique à celui des cellules non irradiées, non exposées aux NDs-H.
- Pour les cellules exposées aux nanoparticules et irradiées, le stress oxydant est triplé par rapport à la référence sans NDs-H mais cette augmentation n'est pas dépendante de la dose de NDs-H.

Il y a donc apport de radicaux libres dans les cellules après incorporation des NDs-H. mais ces radicaux libres induisent une toxicité significative par stress oxydant uniquement après irradiation d'après l'évolution de l'index cellulaire. La génération de radicaux libres est entretenue par les NDs-H puisque même une heure après irradiation, le stress oxydant est plus élevé que sans irradiation, ce qui n'est pas le cas du témoin.

### 4.4. Conclusions

Les nanodiamants ont donc un effet radiosensibilisant qui permet d'amplifier l'effet des radiations en générant une création plus importante de radicaux libres. Une simple exposition à une dose normalement insuffisante pour induire la mort des cellules tumorales permet une mort par stress oxydatif quand les cellules ont été préalablement exposées aux NDs. Les cellules exposées aux NDs peuvent donc être traitées sélectivement. Les NDs sont particulièrement intéressants car ils ne génèrent pas de toxicité en absence de rayonnement et les cellules non irradiées ne seront pas affectées par la présence de NDs. La toxicité initiale est réduite et l'effet radiosensibilisant amplifié pour des NDs-H, qui sont donc particulièrement intéressants.

### Exemple 5 : Utilisation de nanodiamants hydrogénés pour vectoriser des molécules biologiques dans les cellules

Afin de vérifier la capacité des nanodiamants hydrogénés (ND-H) à lier et transporter des molécules d'intérêt biologique dans les cellules, des particules de ND-H ont été mélangées à un volume égal d'une sonde télomérique constituée d'acide nucléique peptidique (ANP, ou *Protein Nucleic acid Analogue, PNA,* en anglais), marquée avec le fluorophore Cy3. Les concentrations finales dans le mélange étaient de 64,52 µg/cm³ pour les ND-H et 0,5645 µM pour la sonde ANP-Cy3. La sonde ANP-Cy3 a été dénaturée par chauffage à 80°C pendant 5 minutes avant d'être mélangée aux ND-H.

Après une incubation de 10 minutes à température ambiante, le mélange de NDs-H et de sonde ANP-Cy3 a été exposé à des cellules Caki-1 en culture (dans des plaques 8 puits Labtek). Les cellules ont été maintenues en présence du mélange pendant 24h et 72h, dans des conditions standard de culture cellulaire. Les noyaux cellulaires ont ensuite été marqués avec un marqueur fluorescent (Hoechst 33342), et les plaques ont été observées directement à l'aide d'un microscope à fluorescence inversé. Pour le marquage nucléaire, le filtre d'excitation/émission était ∼350nm/460nm, et pour le marquage du ANP-Cy3, de 550nm/570nm.

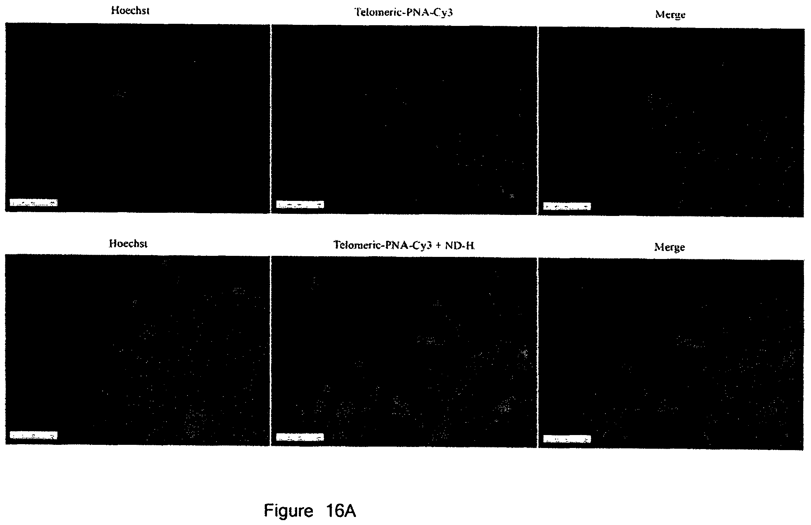

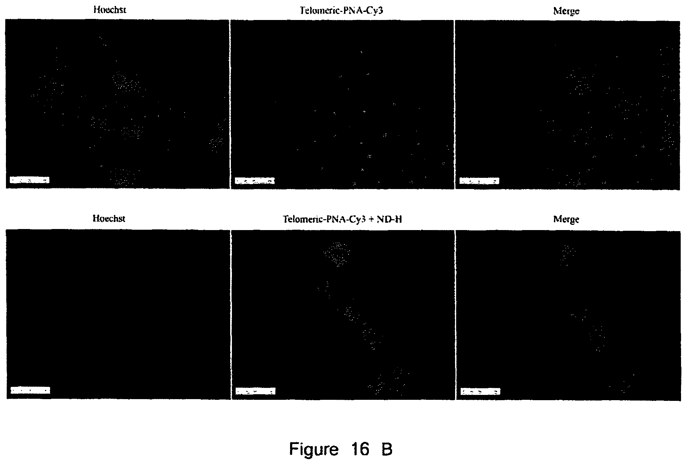

L'internalisation de la sonde de ANP-Cy3 a été observée uniquement dans le cas où les particules de ND-H avaient été mélangées avec cette sonde. En l'absence de ND-H, la sonde ANP-Cy3 n'a pas été internalisée (Figure16).

Ces résultats démontrent la capacité des ND-H à lier une sonde ANP-Cy3 et à la transporter dans les cellules. La molécule internalisée ici n'a pas d'activité cytotoxique majeure, mais elle est chimiquement similaire à des molécules cytotoxiques telles que celle utilisée à l'exemple 6 ci-dessous, qui permettra d'obtenir une synergie avec l'activité cytotoxique des ND-H soumis à des radiations. En outre, cette sonde est moins chargée (négativement) que les molécules cytotoxiques d'intérêt thérapeutique qui pourront être utilisées en clinique. Ces molécules plus chargées négativement s'adsorberont plus facilement à la surface des ND-H et seront plus efficacement vectorisées dans les cellules. Ces résultats démontrent donc la capacité des ND-H à vectoriser à l'intérieur des cellules des molécules cytotoxiques d'intérêt thérapeutique telles que des acides nucléiques ou des ANP.

### Exemple 6 : Utilisation de complexes nanodiamants/ARN interférents radiosensibilisants pour le traitement de tumeurs

Des nanodiamants (taille primaire de 5 nm) sont préparés afin d'avoir un potentiel Zeta positif selon les procédés décrits ci-dessus. Pour les NDs hydrogénés et/ou graphitisés, une étape de sonification dans l'eau permet à la fois de disperser les nanodiamants et d'adsorber efficacement des molécules (H₂O, O₂ et NO₂ principalement) sur leur surface, leur conférant ainsi une charge de surface positive.

Des ARNs interférents POLQ, ayant la capacité d'inhiber les ARN messagers codant pour la polymérase thêta, permettant la réparation de l'ADN dans certaines cellules tumorales (le gêne POLQ est surexprimé dans les cancers du sein les plus agressifs par exemple) sont adsorbés sur la surface des nanodiamants. Les ARNs ayant une charge négative de surface, peuvent être adsorbés par interaction électrostatique sur la surface des nanodiamants chargés positivement par simple addition des ARNs dans la suspension de nanodiamants.

Ces complexes nanodiamants/ARNs sont ensuite injectés dans des cellules tumorales, où ils rentrent préférentiellement du fait de la perméabilité accrue des membranes des cellules tumorales. Les ARNs interférents POLQ sont libérés progressivement dans les cellules tumorales et inhibent la synthèse des polymérases thêta. Les cellules tumorales sont rendues plus sensibles aux radiations.

Les cellules tumorales sont irradiées par des rayons X, entraînant la libération de radicaux libres dans les cellules ayant internalisé les nanodiamants.

### REFERENCES

Arnault, J.-C.; Petit, T.; Girard, H.; Chavanne, A.; Gesset, C.; Sennour, M.; Chaigneau, M. Surface chemical modifications and surface reactivity of nanodiamonds hydrogenated by CVD plasma. Physical Chemistry Chemical Physics, 2011, 13, 11481-11487.
Batsanov, S. S.; Gavrilkin, S. M.; Batsanov, A. S.; Poyarkov, K. B.; Kulakova, I. I..; Johnson D.W. and Mendis B. G., Giant dielectric permittivity of detonation-produced nanodiamond is caused by water, Journal of Materials Chemistry, 2012, 22, 11166-11172.
Begg, A.C., Stewart, F.A. and Vens, C. Strategies to improve radiotherapy with targeted drugs. Nature Review Cancer, 2011, 11, 239-253.
Girard, H. A.; Arnault, J. C. C.; Perruchas, S.; Saada, S.; Gacoin, T.; Boilot, J.-P. P.; Bergonzo, P. Hydrogenation of nanodiamonds using MPCVD: A new route toward organic functionalization. Diamond and Related Materials, 2010, 19, 1117-1123.
Chakrapani, J. C. Angus, A. B. Anderson, S.D. Wolter, B.R. Stoner, G.U. Sumanasekera, Charge Transfer Equilibria Between Diamond and an Aqueous Oxygen Electrochemical Redox Couple, Science, 2007, 318, 1424-30.
Chang, Y.-R.; Lee, H.-Y.; Chen, K.; Chang, C.-C.; Tsai, D.-S.; Fu, C.-C.; Lim, T.-S.; Tzeng, Y.-K.; Fang, C.-Y.; Han, C.-C.; Chang, H.-C. and Fann, W., Mass production and dynamic imaging of fluorescent nanodiamonds, Nature Nanotechnology, 2008, 3, 284-288.
Chao, J.I., Perevedentseva, E., Chung, P.H., Liu, K.K., Cheng, C.Y., Chang, C.C. and Cheng, C,L.,Nanometer-sized diamond particle as a probe for biolabeling. Biophysical Journam, 2007, 93, 2199-2208 (2)
Chen M., Zhang X.-Q., Man H.B., Lam R., Chow E.K., Ho D., Nanodiamond Vectors Functionalized with Polyethylenimine for siRNA Delivery, The Journal of Physical Chemistry Letters, 2010, 1, 3167-3171.
Chen, X.; Zhong, Z.; Xu, Z.; Chen, L.; Wang Y., 2', 7'-Dichlorodihydrofl uorescein as a fluorescent probe for reactive oxygen species measurement: Forty years of application and controversy, Free Radical Research, 2010, 44 (6), 587-604.
Girard, H.A.; Petit, T.; Perruchas, S.; Gacoin, T.; Gesset, C.; Arnault, J.C.; Bergonzo, P. Surface properties of hydrogenated nanodiamonds: a chemical investigation. Physical Chemistry Chemical Physics, 2011, 13, 11517-11523.
Curnis, F., G. Arrigoni, et al., Differential binding of drugs containing the NGR motif to CD13 isoforms in tumor vessels, epithelia, and myeloid cells Cancer Research, 2002, 62(3): 867-74.
Higgins, G.S., Prevo, R., Lee, Y.F., Helleday, T., Muschel, R.J., Taylor, S., Yoshimura, M., Hickson, I.D., Bernhard. E.J. and McKenna, W.G. A small interfering RNA screen of genes involved in DNA repair identifies tumor-specific radiosensitization by POLQ knockdown. Cancer Research, 2010, 70, 2984-2993.
Huang LC, Chang HC. Adsorption and immobilisation of cytochrome c on nanodiamonds. Langmuir, 2004,20 (14),5879-84.
Jarre, G., Liang, Y., Betz, P., Lang, D. and Krueger, A., Playing the surface game-Diels-Alder reactions on diamond nanoparticles. Chemical Communication (Cambridge) ,2011, 47, 544-546.
Krüger A, Liang Y, Jarre G, Stegk J., Surface functionalisation of détonation nanodiamond suitable for biological applications. Journal of Material Chemistry, 2006, 11, 2322-2328.
Petit, T.; Arnault, J.-C.; Girard, H. A.; Sennour, M.; Bergonzo, P. Early stages of surface graphitization on nanodiamond probed by x-ray photoelectron spectroscopy. Physical Review B, 2011, 84, 233407.
Ryu, L. Liu, S. Berciaud, Y.-J. Yu, H. Liu, P. Kim, G.W. Flynn, L.E. Brus, Atmospheric Oxygen Binding and Hole Doping in Deformed Graphene on a SiO2 Substrate, Nano Letters, 2010, 10, 4944-4951.
Yang, K.; Zhang. S.; Zhang, G.; Sun, X.; Lee S.-T. and Liu, Z. Graphene in Mice: Ultrahigh In Vivo Tumor Uptake and Efficient Photothermal Therapy, Nano Letters, 2010, 10, 3318-3323.
Fu Chi-Cheng, Hsu-Yang Lee, Kowa Chen, Tsong-Shin Lim, Hsiao-Yun Wu, Po-Keng Lin, Pei-Kuen Wei, Pei-Hsi Tsao, Huan-Cheng Chang and Wunshain Fann. Characterization and application of single fluorescent nanodiamonds as cellular biomarkers. Proc Natl Acad Sci U S A. 2007 January 16; 104(3): 727-732.
Higgins, G.S., Prevo, R., Lee, Y.F., Helleday, T., Muschel, R.J., Taylor, S., Yoshimura, M., Hickson, I.D., Bernhard, E.J. and McKenna, W.G. A small interfering RNA screen of genes involved in DNA repair identifies tumor-specific radiosensitization by POLQ knockdown. Cancer Research, 2010, 70, 2984-2993.
Huang LC, Chang HC. Adsorption and immobilization of cytochrome c on nanodiamonds. Langmuir, 2004,20 (14),5879-84.
Jarre, G., Liang, Y., Betz, P., Lang, D. and Krueger, A., Playing the surface game-Diels-Alder reactions on diamond nanoparticles. Chemical Communication (Cambridge), 2011, 47, 544-546.
Kuznetsov Oleksandr et al., Water-soluble nanodiamond, Langmuir. The ACS Journal of surfaces and colloids, 2012, 11, 5243-5248.
Krüger A, Liang Y, Jarre G, Stegk J., Surface functionalisation of detonation nanodiamond suitable for biological applications. Journal of Material Chemistry, 2006, 11, 2322-2328.
Petit, T.; Arnault, J.-C.; Girard, H. A.; Sennour, M.; Bergonzo, P. Early stages of surface graphitization on nanodiamond probed by x-ray photoelectron spectroscopy. Physical Review B, 2011, 84, 233407.
Ryu, L. Liu, S. Berciaud, Y.-J. Yu, H. Liu, P. Kim, G. W. Flynn, L. E. Brus, Atmospheric Oxygen Binding and Hole Doping in Deformed Graphene on a SiO2 Substrate, Nano Letters, 2010, 10, 4944-4951.
Yang, K.; Zhang, S.; Zhang, G.; Sun, X.; Lee S.-T. and Liu, Z. Graphene in Mice: Ultrahigh In Vivo Tumor Uptake and Efficient Photothermal Therapy, Nano Letters, 2010, 10,3318-3323.

## Revendications

1. Nanodiamant pour utilisation, en combinaison avec un rayonnement ionisant, comme médicament générant des radicaux libres et destiné à la destruction de cellules cibles.

2. Nanodiamant pour utilisation selon la revendication 1, dans laquelle la génération de radicaux libres est couplé à une génération de chaleur.

3. Nanodiamant pour utilisation selon la revendication 1 ou 2, **caractérisé en ce que** son diamètre moyen est inférieur à 10 nm.

4. Nanodiamant selon l'une quelconque des revendications précédentes, pour utilisation selon la revendication 1 ou 2, **caractérisé en ce que** sa surface a été au moins partiellement graphitisée et/ou hydrogénée.

5. Nanodiamant selon l'une quelconque des revendications précédentes, pour utilisation selon l'une quelconque des revendications précédentes, **caractérisé en ce que** des radicaux libres oxygénés sont générés.

6. Nanodiamant selon l'une quelconque des revendications précédentes, pour utilisation selon l'une quelconque des revendications précédentes, **caractérisé en ce que** des radicaux libres azotés sont générés.

7. Nanodiamant selon l'une quelconque des revendications précédentes, pour utilisation selon l'une quelconque des revendications précédentes, **caractérisé en ce que** le rayonnement ionisant est un rayonnement électromagnétique.

8. Nanodiamant selon l'une quelconque des revendications précédentes, pour utilisation selon l'une quelconque des revendications précédentes, **caractérisé en ce que** le rayonnement ionisant est un rayonnement particulaire.

9. Nanodiamant pour utilisation selon l'une quelconque des revendications précédentes, , **caractérisé en ce que** les cellules cibles sont des cellules cancéreuses.

10. Nanodiamant selon l'une quelconque des revendications précédentes, pour utilisation selon l'une quelconque des revendications précédentes, pour utilisation pour traiter une tumeur solide.

11. Nanodiamant selon l'une quelconque des revendications précédentes, pour utilisation selon l'une quelconque des revendications précédentes, **caractérisé en ce que** le nanodiamant est fonctionnalisé.

12. Nanodiamant selon la revendication 11, **caractérisé en ce qu'**il est lié à une molécule de ciblage.

13. Nanodiamant selon la revendication 12, **caractérisé en ce que** la molécule de ciblage est un ligand biologique reconnu par un récepteur surexprimé à la surface de certaines cellules.

14. Composition comprenant un nanodiamant selon l'une quelconque des revendications précédentes, ainsi qu'une molécule radiosensibilisante liée audit nanodiamant ou adsorbée à sa surface.

15. Composition selon la revendication 14, **caractérisée en ce que** la molécule radiosensibilisante est une molécule d'acide nucléique adsorbée à la surface du nanodiamant.

16. Composition selon la revendication 14, **caractérisée en ce que** la molécule radiosensibilisante est une molécule chimique radiosensibilisante.

17. Composition selon l'une quelconque des revendications 14 à 16, pour utilisation comme médicament, en combinaison avec un rayonnement.

18. Composition pour utilisation selon la revendication 17, **caractérisée en ce que** le rayonnement induit la production de radicaux libres à la surface du nanodiamant.

## Patentansprüche

1. Nanodiamant zur Verwendung, in Kombination mit einer ionisierenden Strahlung, als Arzneimittel, welches freie Radikale erzeugt und zur Zerstörung von Zielzellen bestimmt ist.

2. Nanodiamant zur Verwendung nach Anspruch 1, wobei die Erzeugung von freien Radikalen an eine Erzeugung von Wärme gekoppelt ist.

3. Nanodiamant zur Verwendung nach Anspruch 1 oder 2, **dadurch gekennzeichnet, dass** sein mittlerer Durchmesser kleiner als 10 nm ist.

4. Nanodiamant nach einem der vorstehenden Ansprüche zur Verwendung nach Anspruch 1 oder 2, **dadurch gekennzeichnet, dass** seine Oberfläche mindestens teilweise grafitiert und/oder hydriert wurde.

5. Nanodiamant nach einem der vorstehenden Ansprüche zur Verwendung nach einem der vorstehenden Ansprüche, **dadurch gekennzeichnet, dass** sauerstoffhaltige freie Radikale erzeugt werden.

6. Nanodiamant nach einem der vorstehenden Ansprüche zur Verwendung nach einem der vorstehenden Ansprüche, **dadurch gekennzeichnet, dass** stickstoffhaltige freie Radikale erzeugt werden.

7. Nanodiamant nach einem der vorstehenden Ansprüche zur Verwendung nach einem der vorstehenden Ansprüche, **dadurch gekennzeichnet, dass** die ionisierende Strahlung eine elektromagnetische Strahlung ist.

8. Nanodiamant nach einem der vorstehenden Ansprüche zur Verwendung nach einem der vorstehenden Ansprüche, **dadurch gekennzeichnet, dass** die ionisierende Strahlung eine Teilchenstrahlung ist.

9. Nanodiamant zur Verwendung nach einem der vorstehenden Ansprüche, **dadurch gekennzeichnet, dass** die Zielzellen Krebszellen sind.

10. Nanodiamant nach einem der vorstehenden Ansprüche zur Verwendung nach einem der vorstehenden Ansprüche, zur Verwendung, um einen soliden Tumor zu behandeln.

11. Nanodiamant nach einem der vorstehenden Ansprüche zur Verwendung nach einem der vorstehenden Ansprüche, **dadurch gekennzeichnet, dass** der Nanodiamant funktionalisiert ist.

12. Nanodiamant nach Anspruch 11, **dadurch gekennzeichnet, dass** er an ein Targeting-Molekül gebunden ist.

13. Nanodiamant nach Anspruch 12, **dadurch gekennzeichnet, dass** das Targeting-Molekül ein biologischer Ligand ist, der von einem Rezeptor, welcher an der Oberfläche gewisser Zellen überexprimiert ist, erkannt wird.

14. Zusammensetzung, die einen Nanodiamanten nach einem der vorstehenden Ansprüche sowie ein strahlungssensibilisierendes Molekül umfasst, das an den Nanodiamanten gebunden oder an seine Oberfläche adsorbiert ist.

15. Zusammensetzung nach Anspruch 14, **dadurch gekennzeichnet, dass** das strahlungssensibilisierende Molekül ein Nukleinsäuremolekül ist, das an die Oberfläche des Nanodiamanten adsorbiert ist.

16. Zusammensetzung nach Anspruch 14, **dadurch gekennzeichnet, dass** das strahlungssensibilisierende Molekül ein strahlungssensibilisierendes chemisches Molekül ist.

17. Zusammensetzung nach einem der Ansprüche 14 bis 16 zur Verwendung als Arzneimittel in Kombination mit einer Strahlung.

18. Zusammensetzung zur Verwendung nach Anspruch 17, **dadurch gekennzeichnet, dass** die Strahlung die Produktion von freien Radikalen an der Oberfläche des Nanodiamanten induziert.

## Claims

1. Nanodiamond for use, in combination with an ionising radiation, as a medicament generating free radicals and intended for the destruction of target cells.

2. Nanodiamond for use according to claim 1, wherein the generation of free radicals is coupled with heat generation.

3. Nanodiamond for use according to claim 1 or 2, **characterised in that** the average diameter thereof is less than 10nm.

4. Nanodiamond according to any one of the preceding claims, for use according to claim 1 or 2, **characterised in that** the surface thereof has been at least partially graphitised and/or hydrogenated.

5. Nanodiamond according to any one of the preceding claims, for use according to one of the preceding claims, **characterised in that** oxygenated free radicals are generated.

6. Nanodiamond according to any one of the preceding claims, for use according to one of the preceding claims, **characterised in that** nitrogenated free radicals are generated.

7. Nanodiamond according to any one of the preceding claims, for use according to one of the preceding claims, **characterised in that** the ionising radiation is an electromagnetic radiation.

8. Nanodiamond according to any one of the preceding claims, for use according to any one of the preceding claims, **characterised in that** the ionising radiation is a particle radiation.

9. Nanodiamond for use according to any one of the preceding claims, **characterised in that** the target cells are cancer cells.

10. Nanodiamond according to any one of the preceding claims, for use according to any one of the preceding claims, for use to treat a solid tumour.

11. Nanodiamond according to any one of the preceding claims, for use according to any one of the preceding claims, **characterised in that** the nanodiamond is functionalised.

12. Nanodiamond according to claim 11, **characterised in that** it is bound to a targeting molecule.

13. Nanodiamond according to claim 12, **characterised in that** the targeting molecule is a biological ligand recognised by a receptor overexpressed on the surface of certain cells.

14. Composition comprising a nanodiamond according to any one of the preceding claims, as well as a radiosensitising molecule bound to said nanodiamond or adsorbed on the surface thereof.

15. Composition according to claim 14, **characterised in that** the radiosensitising molecule is a nucleic acid molecule adsorbed on the surface of the nanodiamond.

16. Composition according to claim 14, **characterised in that** the radiosensitising molecule is a radiosensitising chemical molecule.

17. Composition according to any one of claims 14 to 16, for use as a medicament, in combination with a radiation.

18. Composition for use according to claim 17, **characterised in that** the radiation induces the production of free radicals on the surface of the nanodiamond.
